# EUROPÄISCHE PATENTSCHRIFT

(11) **EP 3 685 164 B1**
(45) Veröffentlichungstag und Bekanntmachung des Hinweises auf die Patenterteilung: **26.08.2026**
(21) Anmeldenummer: 18778839.3
(22) Anmeldetag: 17.09.2018
(51) Int. Cl.: G01N 33/50, C12M 1/00

(54) **MIKROFLUIDISCHE VORRICHTUNG FÜR ZELLKULTUREXPERIMENTE UND VERWENDUNGEN HIERVON**
MICROFLUIDIC DEVICE FOR CELL CULTURE EXPERIMENTS AND USES THEREOF
DISPOSITIF MICROFLUIDIQUE POUR DES EXPÉRIENCES DE CULTURE CELLULAIRE ET UTILISATIONS DE CELUI-CI

(30) Priorität: 19.09.2017 DE 102017216581
(43) Veröffentlichungstag der Anmeldung: 29.07.2020
(73) Patentinhaber: Fraunhofer-Gesellschaft zur Förderung der angewandten Forschung e.V., 80686 München (DE); Technische Universität Dresden, 01069 Dresden (DE)
(72) Erfinder: LOSKILL, Peter, 70199 Stuttgart (DE); PROBST, Christopher, 70186 Stuttgart (DE); BUSEK, Mathias, 01277 Dresden (DE); GRÜNZNER, Stefan, 01159 Dresden (DE); SONNTAG, Frank, 01277 Dresden (DE)
(74) Vertreter: Pfenning, Meinig & Partner mbB
(86) Internationale Anmeldenummer: PCT/EP2018/075090
(87) Internationale Veröffentlichungsnummer: WO 2019/057671

(56) Entgegenhaltungen:
- CN-A- 106 811 411
- DE-A1- 102013 011 768
- US-A1- 2012 034 695
- SAEED RISMANI YAZDI ET AL: "Adding the 'heart' to hanging drop networks for microphysiological multi-tissue experiments", LAB ON A CHIP, vol. 15, no. 21, 1 January 2015 (2015-01-01), pages 4138 - 4147, XP055519067, ISSN: 1473-0197, DOI: 10.1039/C5LC01000D
- AEREAS AUNG ET AL: "3D cardiac [mu]tissues within a microfluidic device with real-time contractile stress readout", LAB ON A CHIP, vol. 16, no. 1, 1 January 2016 (2016-01-01), pages 153 - 162, XP055518460, ISSN: 1473-0197, DOI: 10.1039/C5LC00820D
- ANA PERESTRELO ET AL: "Microfluidic Organ/Body-on-a-Chip Devices at the Convergence of Biology and Microengineering", SENSORS, vol. 15, no. 12, 10 December 2015 (2015-12-10), pages 31142 - 31170, XP055413925, DOI: 10.3390/s151229848

## Beschreibung

Erfindungsgemäß wird eine mikrofluidische Vorrichtung vorgestellt, die mindestens eine erste Kulturkammer mit Herzmuskelzellen, mindestens einen mikrofluidischen Kanal, mindestens eine Pumpe (z.B. eine Mikropumpe) und mindestens einen Detektor aufweist, wobei der Detektor zur Erfassung einer Aktivität der in der Kulturkammer enthaltenen Herzmuskelzellen konfiguriert ist. Die mikrofluidische Vorrichtung enthält ferner mindestens eine Steuereinrichtung, die dazu konfiguriert ist, die mindestens eine Pumpe basierend auf einer von dem mindestens einen Detektor erfassten Aktivität der Herzmuskelzellen zu steuern. Ferner werden Verwendungen der mikrofluidischen Vorrichtung vorgeschlagen.

Weltweit beschäftigen sich viele Arbeitsgruppen mit der Entwicklung von artifiziellem Herzgewebe. Insbesondere für die tierversuchsfreie Substanztestung werden hier neue Impulse erwartet, da mit zellbasierten Systemen eine patientennahe und individualisierte Wirkstofffindung möglich ist. Bisherige in-vitro-Plattformen sind meist zu einfach aufgebaut (2-dimensional, keine Perfusion, fehlendes Mikromilieu), um das physiologische Verhalten von Herzgewebe ausreichend nachzustellen. Es besteht somit ein Bedarf an mikrofluidischen Vorrichtungen, auch mikrophysiologische Systeme (kurz: "MPS") genannt, die das Mikromilieu nachbilden und 3-dimensionale Geweberäume bieten, die dem menschlichen Gewebe näher kommen. Ziel ist einerseits der möglichst in-vivo-nahe Aufbau von Organmodellen und andererseits die Kultivierung mehrerer solcher Modelle in einem geschlossenen Kreislaufsystem, um daran systemische Wirkstofftestung durchführen zu können (sog. "Mehrorganchip").

Unter physiologischen Bedingungen beginnen die Herzmuskelzellen mit spontaner oder (elektrisch) stimulierter Bewegung, welche durch das vorherrschende Mikromilieu (Hypoxie, Nährstoffmangel), Stoffwechselprodukte oder die Zugabe von Substanzen beeinflusst werden kann. Schlagfrequenz, Kontraktionsgeschwindigkeit (bzw. Relaxationsgeschwindigkeit), Kontraktionskraft, Kontraktionsdauer, Rhythmizität und maximale Verformung sind dabei pharmakologisch relevante Parameter, welche z.B. durch mikroskopische Beobachtung, beispielsweise mit anschließender Videoanalyse, ermittelt werden können. Der menschliche Körper besitzt ausgehend von diesen Parametern einen komplexen Regelmechanismus, welcher in bisherigen MPS fehlt. Beispielsweise können bisherige mikrofluidische Vorrichtungen nicht abbilden, dass das Herz als Selbstversorger bei sinkender Förderleistung selbst auch schlechter versorgt wird (was wiederum ein weiteres Absinken der Förderleitung zur Folge haben kann).

Im Stand der Technik sind bislang eine Reihe unterschiedlicher Herzmuskel in-vitro-Systeme bekannt, die sich in Größe (Einzelzellen bis zu makroskopischen 3D-Geweben), Ursprung (z.B. primäre Nagerzellen, Zelllinien, basierend auf Stammzellen), Struktur (isotrop oder anisotrop) und auch der Implementierung der Perfusion unterscheiden. Die Nachbildung des Herzmuskels ist dabei in den meisten Fällen fokussiert auf dessen Verwendung als "elektromechanischer Sensor", um die Auswirkungen von Arzneimitteln oder Stimuli auf die Gewebe direkt zu ermitteln. In nur wenigen Studien wurden die nachgebildeten Herzmuskelgewebe tatsächlich zur Erzeugung einer Perfusion verwendet. Saeed Rismani Yazdi et al.: "Adding the 'heart' to hanging drop networks for microphysiological multi-tissue experiments",LAB ON A CHIP, Bd. 15, Nr. 21, 1. Januar 2015 (2015-01-01), Seiten 4138-4147, offenbart mikrofluidische Vorrichtungen enthaltend Pumpen, Kulturkammern mit Herzmuskelzellen und Detektoren, wobei die Pumpgeschwindigkeit aufgrund der vom Detektor erfassten Herzmuskelaktivität reguliert wird.

Aufgrund der geringen Kräfte, die die Mikroherzmuskeln erzeugen, ist eine direkte Erzeugung der Perfusion (Kardiomyozythenpumpe) aufgrund des hohen fluidischen Widerstands von Mikrokanälen nicht praktikabel.

Das Auslesen von Kontraktionsbewegungen bzw. der Kontraktionskräfte ist in den Herzmuskel-in-vitro-Systemen im Stand der Technik auf unterschiedliche Weise realisiert. Beispielsweise werden hierfür flexible Federbalken, 3D-gedruckte Dehnmessstreifen und auch optische Analysemethoden verwendet. Darüber hinaus kommen elektrophysiologische Sensoren, wie beispielsweise Mikroelektroden-Arrays (MEA), Patch-Clamp, "sharp electrodes" und Calcium-Bildgebung zum Einsatz.

Aus der DE 10 2013 011 768 A1 ist ein Zirkulationssystem bekannt und es wird ein Verfahren zur Vitalversorgung von Zellkulturen in einem mikrofluidischen Netzwerk vorgestellt. In diesem mikrofluidischen Netzwerk erfolgt eine definierte Versorgung mehrerer Zellkultur-Kompartimente durch Drosseln und/oder Ventile. Mit diesen Drosseln und/oder Ventilen kann beispielsweise der pH-Wert, der Sauerstoffpartialdruck, der Kohlendioxidpartialdruck und/oder die Glukose-Konzentration eingestellt und geregelt werden.

Auch mehrlagige mikrofluidische Vorrichtungen mit integrierten aktiven Komponenten (wie beispielsweise Pumpen und Oxygenatoren) zur direkten Kultivierung von Herzmuskelzellen konnten bereits realisiert werden.

Weiterhin ist ein Modul mit einer Feedback-Schleife zur Regelung und Einstellung definierter Gaszusammensetzungen im Stand der Technik bekannt.

Bislang zeichnen sich die im Stand der Technik bekannten MPS mit Herzmuskelzellen durch den Nachteil aus, dass sie den Einfluss der Aktivität von Herzmuskelzellen auf den Fluidstrom in der mikrofluidischen Vorrichtung gar nicht bis lediglich unzureichend abbilden. Anders ausgedrückt erlauben es die bekannten Systeme bzw. Vorrichtungen nicht, eine bestimmte zu einem bestimmten Zeitpunkt vorliegende Aktivität der Herzmuskelzellen praktisch unmittelbar (d.h. ohne zeitliche Verzögerung) in einen der vorliegenden Aktivität entsprechenden Fluidstrom in dem System zu übersetzen, wie dieses in-vivo durch den Regelmechanismus des Herz-Kreislaufsystems durchaus vermittelt wird. Als Folge dieses Nachteils kann mit derzeit bekannten mikrofluidischen Vorrichtungen mit Herzzellen der echte in-vivo-Zustand eines lebenden Patienten nur unzureichend simuliert werden, da sich beim lebenden Patienten eine Änderung der Aktivität der Herzmuskelzellen mitunter sehr stark auf den Fluidstrom des Blutkreislaufs und damit die Blut- und Sauerstoffversorgung einzelner Zellen und Organe auswirken kann.

Ausgehend hiervon war die Aufgabe der vorliegenden Erfindung, eine mikrofluidische Vorrichtung mit Herzmuskelzellen bereitzustellen, die eine genauere und realitätsnähere Untersuchung des Einflusses bestimmter Faktoren auf die Aktivität von Herzmuskelzellen erlaubt und zudem ermöglicht, die Aktivität anderer biologischer Zellen (z.B. Leberzellen oder Nervenzellen) in Abhängigkeit von der geänderten Herzmuskelzellaktivität möglichst realitätsnah zu erfassen (z.B. unter den Bedingungen einer Arrhythmie oder eines kardiogenen Schocks).

Die Aufgabe wird gelöst durch die mikrofluidische Vorrichtung mit den Merkmalen von Anspruch 1 und die Verwendungen gemäß Anspruch 15. Die abhängigen Ansprüche zeigen vorteilhafte Weiterbildungen auf.

Erfindungsgemäß wird eine mikrofluidische Vorrichtung bereitgestellt, enthaltend
a) mindestens eine erste Kulturkammer enthaltend Herzmuskelzellen;
b) mindestens einen mikrofluidischen Kanal;
c) mindestens eine Pumpe (z.B. eine Mikropumpe) zur Beförderung einer Flüssigkeit durch den mindestens einen mikrofluidischen Kanal und die mindestens eine Kulturkammer;
d) mindestens einen Detektor, der zur Erfassung einer Aktivität der in der Kulturkammer enthaltenen Herzmuskelzellen konfiguriert ist;

wobei die mikrofluidische Vorrichtung ferner mindestens eine Steuereinrichtung enthält, die dazu konfiguriert ist, die mindestens eine Pumpe basierend auf einer von dem mindestens einen Detektor erfassten Aktivität der Herzmuskelzellen zu steuern,
wobei die mikrofluidische Vorrichtung mindestens eine Vorrichtung zur Beeinflussung einer Aktivität der in der Kulturkammer enthaltenen Herzmuskelzellen enthält,
dadurch gekennzeichnet, dass die Vorrichtung zur Beeinflussung einer Aktivität der in der Kulturkammer enthaltenen Herzmuskelzellen eine Elektrode enthält.

Unter dem Begriff "Aktivität der Herzmuskelzellen" wird insbesondere eine elektrische Aktivität und/oder Bewegungsaktivität der Herzmuskelzellen (bevorzugt beides) verstanden, wobei der Begriff auch eine elektrische Aktivität von Null (kein Aktionspotential) und eine Bewegungsaktivität von Null (Bewegungsstillstand) umfasst. Unter der Bewegungsaktivität der Herzmuskelzellen wird insbesondere die Kontraktilität und/oder die Bewegungsfrequenz der Herzmuskelzellen (bevorzugt beides) verstanden.

In der erfindungsgemäßen Vorrichtung kann die Aktivität von Herzmuskelzellen erfasst werden und über eine Rückkopplungsschleife (engl. "feedback loop") zur Pumpe die geförderte Fluidmenge (Perfusion) entsprechend der Aktivität der Herzmuskelzellen angepasst werden. Durch diese Maßnahme kann nicht nur ein Einfluss einer geänderten Perfusion auf die Herzmuskelzellen selbst realitätsnäher studiert werden, sondern auch ein Einfluss der geänderten Perfusion auf andere biologischen Zellen, die sich in weiteren Kulturkammern in der mikrofluidischen Vorrichtung befinden.

Durch die Rückkopplungsschleife kann der komplexe Regelmechanismus des Herz-Kreislaufsystems von lebenden Organismen besser nachgebildet werden als bei bisherigen Systemen bzw. Vorrichtungen aus dem Stand der Technik. Anders ausgedrückt ist es mit der erfindungsgemäßen Vorrichtung möglich, eine realitätsnähere und in Bezug auf die wahre in-vivo-Situation relevantere Aussagen zu treffen, welchen Einfluss bestimmte Faktoren auf das Herz-Kreislaufsystem haben. Durch die prinzipielle Möglichkeit, Flüssigkeit in der mikrofluidischen Vorrichtung in einem Kreislauf zu führen ist zudem die Möglichkeit eröffnet, ein Risiko von zeitlich verzögerten Folgeerkrankungen treffender vorherzusagen, da bestimmte Substanzen bzw. Stoffwechselprodukte über einen langen Zeitraum wirken können.

Ferner kann durch die erfindungsgemäße Vorrichtung realitätsnah die Wirkung einer veränderten Aktivität von Herzmuskelzellen auf weitere Gewebetypen (z.B. Lebergewebe, Nervenzellen usw.) nachgebildet werden, was insbesondere für eine systemische Testung von (z.B. chemischen, biochemischen oder biologischen) Substanzen von entscheidender Bedeutung ist.

Die mikrofluidische Vorrichtung kann dadurch gekennzeichnet sein, dass die mindestens eine Steuereinrichtung dazu konfiguriert ist, bei einer starken Aktivität der Herzmuskelzellen den Pumpendurchsatz der mindestens einen Pumpe zu erhöhen, bevorzugt über eine Erhöhung der Pumpfrequenz und/oder des Pumpenhubs. Ferner kann die mindestens eine Steuereinrichtung dazu konfiguriert sein, bei einer schwachen Aktivität der Herzmuskelzellen den Pumpendurchsatz der mindestens einen Pumpe zu verringern, bevorzugt über eine Verringerung der Pumpfrequenz und/oder des Pumpenhubs.

Die mindestens eine Steuereinrichtung kann ferner konfiguriert sein, eine Perfusion der mindestens einen ersten Kulturkammer enthaltend Herzmuskelzellen zu verringern oder zu erhöhen, bevorzugt über ein Öffnen oder Schließen eines zur mindestens einen ersten Kulturkammer fluidisch parallel geschalteten Kurzschlusskanals. Darüberhinaus kann die mindestens eine Steuereinrichtung konfiguriert sein, eine Perfusion mindestens einer zweiten Kulturkammer in der mikrofluidischen Vorrichtung zu verringern oder zu erhöhen, bevorzugt über ein Öffnen oder Schließen eines zur mindestens einen zweiten Kulturkammer fluidisch parallel geschalteten Kurzschlusskanals.

Die mindestens eine erste Kulturkammer kann Herzmuskelzellen enthalten, die mindestens eine Herzmuskelfaser ausbilden, wobei die Herzmuskelzellen der mindestens einen Herzmuskelfaser bevorzugt anisotrop ausgerichtet sind. Ferner können die Kulturkammern biologische Zellen enthalten, die sich von Herzmuskelzellen unterscheiden, bevorzugt Zellen ausgewählt aus der Gruppe bestehend aus Fibroblasten, Endothelzellen und Kombinationen hiervon. Darüber hinaus können die Herzmuskelzellen ein Hydrogel enthalten, bzw. in diesem eingebettet sein.

In einer bevorzugten Ausgestaltungsform weist die mikrofluidische Vorrichtung mindestens ein Reservoir auf, das eine Nährlösung zur Ernährung von Herzmuskelzellen enthält, wobei das Reservoir bevorzugt fluidisch mit dem mindestens einen mikrofluidischen Kanal und der mindestens einen Kulturkammer verbunden ist und besonders bevorzugt die mindestens eine Pumpe dazu konfiguriert ist, die Nährlösung zur mindestens einen Kulturkammer zu befördern, insbesondere über den mindestens einen mikrofluidischen Kanal.

Die mikrofluidische Vorrichtung kann dazu konfiguriert sein, bei einer starken Aktivität der Herzmuskelzellen den Austritt von Nährlösung aus dem Reservoir zu erhöhen, bevorzugt über eine Erhöhung der Pumpfrequenz und/oder des Pumphubs und/oder durch Druckbeaufschlagung des Reservoirs Ferner kann die mikrofluidische Vorrichtung dazu konfiguriert sein, bei einer schwachen Aktivität der Herzmuskelzellen den Austritt von Nährlösung aus dem Reservoir zu verringern, bevorzugt über eine Verringerung der Pumpfrequenz und/oder des Pumphubs und/oder durch Druckerniedrigung am Reservoir.

Der mikrofluidische Kanal kann mindestens ein Ventil und/oder mindestens eine Drossel enthalten. Das mindestens eine Ventil und/oder die mindestens eine Drossel enthalten bevorzugt eine elastische Membran, die bevorzugt in mindestens einer Wandung des mindestens einen mikrofluidischen Kanals angeordnet ist und besonders bevorzugt Kunststoff enthält oder daraus besteht, ganz besonders bevorzugt einen Kunststoff ausgewählt aus der Gruppe bestehend aus Duroplast, Thermoplast, Elastomer und Kombinationen hiervon, insbesondere einen Kunststoff ausgewählt aus der Gruppe bestehend aus PC, PET, COC, PDMS, TPE und Kombinationen hiervon. Ferner kann die elastische Membran dazu geeignet sein, über eine pneumatische, thermopneumatische, elektromagnetische, elektrostatische, magnetische, chemische und/oder piezoelektrische Kraft gesteuert zu werden, wobei das Ventil und/oder die Drossel bevorzugt mit mindestens einem Aktuierungskanal (z.B. einem Pneumatikkanal und/oder einem Hydraulikkanal) und/oder mindestens einer Spannungsquelle verbunden ist.

Die mindestens eine Steuereinrichtung kann konfiguriert sein, basierend auf einer von dem mindestens einen Detektor erfassten Aktivität der Herzmuskelzellen, das mindestens eine Ventil und/oder die mindestens eine Drossel zu steuern. In diesem Fall ist die mindestens eine Steuereinrichtung bevorzugt dazu konfiguriert, bei einer starken Aktivität der Herzmuskelzellen das mindestens eine Ventil und/oder die mindestens eine Drossel zumindest teilweise zu öffnen. Ferner kann die mindestens eine Steuervorrichtung dazu konfiguriert sein, bei einer schwachen Aktivität der Herzmuskelzellen das mindestens eine Ventil und/oder die mindestens eine Drossel zumindest teilweise zu schließen.

Die mikrofluidische Vorrichtung kann mindestens einen Oxygenator zur Oxygenierung oder Deoxygenierung der Flüssigkeit in der mikrofluidische Vorrichtung, bevorzugt eine gaspermeable Membran und/oder eine Hohlfaser, enthalten. Ferner kann die mikrofluidische Vorrichtung einen O₂-Sensor zur Messung des O₂-Gehalts der Flüssigkeit in der mikrofluidischen Vorrichtung, bevorzugt einen O₂-Sensor ausgewählt aus der Gruppe bestehend aus optischer O₂-Sensor, elektrochemischer O₂-Sensor und Kombinationen hiervon, enthalten.

Die mindestens eine Steuereinrichtung kann ferner konfiguriert sein, den Oxygenator in Abhängigkeit von einem über den O₂-Sensor gemessenen O₂-Gehalt und/oder in Abhängigkeit von der vom mindestens einen Detektor erfassten Aktivität der Herzmuskelzellen zu steuern.

In einer bevorzugten Ausgestaltungsform enthält der Detektor einen optischen Detektor, bevorzugt einen zur Messung einer Calcium-Konzentration konfigurierten optischen Detektor, oder besteht daraus. Im Falle eines optischen Detektors ist die Kulturkammer der mikrofluidischen Vorrichtung zumindest bereichsweise für Licht der Wellenlänge des optischen Detektors (bevorzugt VIS-Spektrum) transparent. Beispielsweise kann der optische Detektor eine Mikroskopkamera enthalten, die insbesondere mit einer echtzeitfähigen Auswerteeinheit gekoppelt ist, wobei die Auswerteeinheit bevorzugt dazu konfiguriert ist, pharmakologisch relevante Parameter zu ermitteln.

Ferner kann der Detektor einen elektrischen Detektor, bevorzugt ein Multi-Elektroden-Array (MEA) enthalten oder daraus bestehen. Die elektrischen Sensoren können eine Laufzeitmessung durchführen. Vorteilhaft an einer nicht-optischen Detektion - ist ein geringerer Hardwareaufwand bei der Bildauswertung. Darüber hinaus kann der Detektor einen mechanischen Detektor, bevorzugt einen Detektor ausgewählt aus der Gruppe bestehend aus Dehnmessstreifen, Auflagekraft-Mikroskop, Federbalken und Kombinationen hiervon enthalten oder daraus bestehen. In gewisser Hinsicht kann die mikrofluidische Vorrichtung im Falle dieser Detektoren als zellbasierter (chemo- )(elektro-)(opto-)mechanischer Wandler angesehen werden, der auf Änderung des Mikromilieus, Stoffwechselprodukte oder Substanzbeaufschlagung reagiert.

Der Detektor kann dazu konfiguriert sein, Signale über eine Aktivität der in der mindestens einen ersten Kulturkammer enthaltenen Herzmuskelzellen an ein Datenerfassungsgerät zu senden, wobei das Datenerfassungsgerät bevorzugt dazu konfiguriert ist, die Signale in Abhängigkeit von der Zeit aufzuzeichnen und auszuwerten.

Erfindungsgemäß enthält die mikrofluidische Vorrichtung mindestens eine Vorrichtung zur Beeinflussung einer Aktivität der in der mindestens einen ersten Kulturkammer enthaltenen Herzmuskelzellen. Dadurch können Krankheiten des Herz-Kreislaufsystems, wie z.B. Herzinsuffizienz, Herzrhythmusstörungen, etc. künstlich erzeugt werden.

Die Vorrichtung zur Beeinflussung der Aktivität der Herzmuskelzellen enthält erfindungsgemäß eine Elektrode, besonders bevorzugt mindestens eine Elektrode ausgewählt aus der Gruppe bestehend aus Eintauchelektrode, planare Elektrode oder eine Kombination hiervon, oder besteht daraus. Insbesondere enthält die Vorrichtung ein Multi-Elektroden-Array oder besteht daraus. Eine elektrische Kontaktierung der Herzmuskelzellen über diese Vorrichtung bietet die Möglichkeit, das Herzgewebe elektrisch zu stimulieren, z.B. um bestimmte Zustände wie körperliche Belastung nachzubilden. Der Vorteil von Multi-Elektrodenarrays (MEA) ist, dass sie zudem als elektrische Sensoren dienen können, also Stimulation der Herzmuskelzellen und Erfassung ihrer Aktivität gleichermaßen durchführen können.

Ferner kann die Vorrichtung zur Beeinflussung einer Aktivität der in der mindestens einen ersten Kulturkammer enthaltenen Herzmuskelzellen mindestens einen Aktor, besonders bevorzugt einen Aktor ausgewählt aus der Gruppe bestehend aus piezoelektrische Aktoren, elektromechanische Aktoren, pneumatische Aktoren, hydraulischen Aktoren, Oberflächenspannungsaktoren und Kombinationen hiervon, enthalten.

Darüber hinaus kann die Vorrichtung zur Beeinflussung einer Aktivität der in der Kulturkammer enthaltenen Herzmuskelzellen eine Vorrichtung zur Zuführung oder Abführung von Gas, bevorzugt eine Gasaustauschmembran, enthalten, wobei die Vorrichtung zur Zuführung oder Abführung von Gas bevorzugt eine Quelle eines Gases ausgewählt aus der Gruppe bestehend aus (reinem) Sauerstoff, Luft (z.B. Druckluft), Stickstoff, Kohlendioxid und Kombinationen hiervon aufweist, insbesondere eine Quelle eines Gases ausgewählt aus der Gruppe bestehend aus Luft (z.B. Druckluft), Stickstoff, Kohlendioxid und Kombinationen hiervon.

In einer besonders bevorzugten Ausgestaltungsform enthält die mikrofluidische Vorrichtung mindestens einen Zugang zur Zuführung einer (z.B. chemischen und /oder biologischen und/oder biochemischen) Substanz. Diese Ausgestaltungsform ist für pharmazeutische Wirkstofftestungen von Vorteil. Optional mündet dieser Zugang unmittelbar in den mindestens einen mikrofluidischen Kanal, unmittelbar in die mindestens eine erste Kulturkammer und/oder unmittelbar in das mindestens eine Reservoir. Die Beaufschlagung mit einer oder mehreren chemischen, biochemischen oder biologischen Substanzen kann somit direkt auf das Gewebe oder indirekt (z.B. durch die Ein- und Auslässe der mikrofluidischen Vorrichtung) über das strömende Medium erfolgen.

Die mikrofluidische Vorrichtung kann mindestens einen zweiten mikrofluidischen Kanal enthalten, der bevorzugt stromaufwärts der mindestens einen ersten Kulturkammer von dem mindestens einen ersten mikrofluidischen Kanal abzweigt und stromabwärts der mindestens einen ersten Kulturkammer in den mindestens einen ersten mikrofluidischen Kanal mündet.

Ferner kann die mikrofluidische Vorrichtung mindestens eine zweite Kulturkammer aufweisen, die biologische Zellen enthält, welche sich von Herzmuskelzellen unterscheiden. Bevorzugt sind diese Zellen ausgewählt aus der Gruppe bestehend aus Leberzellen, Nierenzellen, Nervenzellen, Fettgewebe und Kombinationen hiervon, wobei die mindestens eine zweite Kulturkammer besonders bevorzugt mit der mindestens einen Kulturkammer fluidisch verbunden ist, optional über mindestens einen zweiten mikrofluidischen Kanal. Stromaufwärts der zweiten Kulturkammer kann eine Drossel oder ein Ventil für die Strömungsbeeinflussung enthalten sein. Die mikrofluidische Vorrichtung kann ferner mehr als zwei solcher Kulturkammern aufweisen, wobei jede der Kulturkammern diese Eigenschaften aufweisen kann. Die Kulturkammern können auch jeweils als separate untergeordnete mikrofluidische Vorrichtungen ausgestaltet sein, die mit einem mikrofluidischen Kanal der übergeordneten mikrofluidischen Vorrichtung , z.B. über eine Steckverbindung, verbunden sind.

Die mikrofluidische Vorrichtung kann mindestens einen zweiten Detektor enthalten, der zur Erfassung einer Aktivität der in der zweiten Kulturkammer enthaltenen biologischen Zellen konfiguriert ist. Der mindestens eine zweite Detektor enthält bevorzugt einen optischen Detektor oder besteht daraus und ist besonders bevorzugt dazu konfiguriert, Signale über eine Aktivität der in der mindestens einen zweiten Kulturkammer enthaltenen biologischen Zellen an ein Datenerfassungsgerät zu senden, wobei das Datenerfassungsgerät bevorzugt dazu konfiguriert ist, die Signale in Abhängigkeit von der Zeit aufzuzeichnen und auszuwerten.

In einer bevorzugten Ausgestaltungsform enthält die mikrofluidische Vorrichtung, bevorzugt der mindestens eine mikrofluidische Kanal, die mindestens eine erste Kulturkammer und/oder mindestens ein Reservoir, Blut oder Bestandteile von Blut, bevorzugt Blutzellen, besonders bevorzugt Krebszellen und/oder Immunzellen, insbesondere metastasierende Krebszellen und/oder Monocyten.

Die mindestens eine Steuereinrichtung kann eine Flussgeschwindigkeits-Messvorrichtung enthalten, bevorzugt ein Particle-Image-Velocimetry-Gerät, wobei die mikrofluidische Vorrichtung besonders bevorzugt konfiguriert ist, anhand einer von der Flussgeschwindigkeits-Messvorrichtung gemessenen Flussgeschwindigkeit die mindestens eine Pumpe, bevorzugt alle Pumpen, der mikrofluidische Vorrichtung zu steuern. Ferner kann die mikrofluidische Vorrichtung besonders bevorzugt konfiguriert sein, anhand einer von der Flussgeschwindigkeits-Messvorrichtung gemessenen Flussgeschwindigkeit mindestens ein Ventil und/oder mindestens eine Drossel, bevorzugt alle Ventile und/oder alle Drosseln, der mikrofluidischen Vorrichtung, zu steuern.

Die mikrofluidische Vorrichtung kann Kunststoff enthalten oder daraus bestehen. Der Kunststoff enthält bevorzugt mindestens eine strukturierte Kunststofffolie, die mit mindestens einer weiteren, optional unstrukturierten, Kunststofffolie laminiert ist, oder besteht daraus. Ferner kann der Kunststoff ausgewählt sein aus der Gruppe bestehend aus Duroplasten, Thermoplasten, Elastomeren und Kombinationen hiervon, besonders bevorzugt aus einem Kunststoff ausgewählt aus der Gruppe bestehend aus PC, PET, COC, PDMS, TPE und Kombinationen hiervon. Darüber hinaus kann der Kunststoff zumindest teilweise über ein Verfahren strukturiert sein, das ausgewählt ist aus der Gruppe bestehend aus Laserstrukturierung, Schneidplottern, Heißprägen, Fräsen, Thermoformen, Spritzguss, Softlithografie, 3D-Druck und Kombinationen hiervon.

Die Steuereinrichtung kann konfiguriert sein, eine Verteilung von flüssigen, festen und gasförmigen Stoffen in der mikrofluidischen Vorrichtung an einen konkreten Bedarf von Zellen oder Zellgruppen in der mikrofluidische Vorrichtung anzupassen, bevorzugt über eine selektive Ansteuerung von der Pumpe, von mindestens einem Ventil, von mindestens einer Drossel und von einem Oxygenator, wobei die selektive Ansteuerung besonders bevorzugt über ein mathematisches Modell erfolgt, das insbesondere in der mikrofluidischen Vorrichtung, bevorzugt in der Steuereinrichtung, gespeichert ist. Das mathematische Modell enthält dabei eine physikalische Beschreibung des Strömungs- und Stofftransports in der unter-oder übergeordneten mikrofluidischen Vorrichtung bzw. in der gesamten mikrofluidischen Vorrichtung.

Die mikrofluidische Vorrichtung kann ferner eine Vorrichtung zur Erfassung einer Strömungsgeschwindigkeit von Fluiden enthalten. Dadurch kann die tatsächliche Strömungsgeschwindigkeit des Fluids überwacht werden. Die Erfassung der Strömungsgeschwindigkeit kann dabei auf nicht-invasive Art und Weise an strömenden Partikeln oder Zellen (z.B. Blutbestandteilen) mittels PIV mit einem adaptierten Motion-Tracking Modul erfolgen. Möglich ist aber auch eine invasive Messung (z.B. durch ein thermisches Anemometer).

Ferner kann die mikrofluidische Vorrichtung eine Vorrichtung zur Erfassung des O₂-Gehalts in Fluiden sowie eine Gasquelle zur Steuerung des O₂-Gehalts in Fluiden enthalten. Dadurch kann falls nötig eine Anreicherung von Flüssigkeit in der mikrofluidischen Vorrichtung mit Sauerstoff erfolgen.

Die mikrofluidische Vorrichtung kann mindestens eine weitere mikrofluidische Vorrichtung aufweisen, die fluidisch parallel oder seriell mit der (ersten) mikrofluidischen Vorrichtung verbunden ist (z.B. über nur eine einzelne fluidische Verbindung, d.h. einen einzelnen mikrofluidischen Kanal). Die fluidische Verbindung weist bevorzugt mindestens einen fluidischen Einlass und mindestens einen fluidischen Auslass auf (z.B. jeweils in Form von mikrofluidischen Kanälen). Es kann daher zwischen der (ersten) mikrofluidischen Vorrichtung und der weiteren mikrofluidischen Vorrichtung ein Fluidkreislauf gebildet werden. Hierbei kann die (erste) mikrofluidische Vorrichtung eine übergeordnete mikrofluidische Vorrichtung sein und die mindestens eine weitere fluidische Vorrichtung eine untergeordnete mikrofluidische Vorrichtung sein (und *vice versa*). Darüberhinaus kann sich die erfindungsgemäße mikrofluidische Vorrichtung selbst in beispielsweise einen untergeordneten Teil und einen übergeordneten Teil aufspalten. Hierbei kann die mindestens eine erste Kulturkammer enthaltend Herzmuskelzellen in einem untergeordneten Teil der mikrofluidischen Vorrichtung enthalten sein und beispielsweise die mindestens eine Pumpe und/oder der mindestens einen Detektor in einem übergeordneten Teil der mikrofluidischen Vorrichtung. Falls in dem übergeordneten Teil der mindestens eine Detektor angeordnet ist, kann dieser mit dem untergeordneten Teil fluidisch verbunden sein. Im Falle eines optischen Detektors ist jedoch diese fluidische Verbindung nicht nötig. Ein übergeordneter Teil der mikrofluidischen Vorrichtung bzw. eine übergeordnete, weitere mikrofluidische Vorrichtung kann in Form einer Schicht ausgestaltet sein, die einen untergeordneten Teil der mikrofluidischen Vorrichtung bzw. eine untergeordnete, weitere mikrofluidische Vorrichtung kontaktiert (und *vice versa*)*.*

Ferner kann die mikrofluidische Vorrichtung mindestens zwei weitere mikrofluidische Vorrichtungen aufweisen, die fluidisch parallel oder seriell mit der (ersten) mikrofluidischen Vorrichtung und/oder untereinander verbunden sind (z.B. jeweils über nur eine einzelne fluidische Verbindung, d.h. einen einzelnen mikrofluidischen Kanal). Die fluidische Verbindung weist bevorzugt mindestens einen fluidischen Einlass und mindestens einen fluidischen Auslass auf (z.B. jeweils in Form von mikrofluidischen Kanälen). Es kann daher zwischen der (ersten) mikrofluidischen Vorrichtung und den mindestens zwei weiteren mikrofluidischen Vorrichtungen ein Fluidkreislauf gebildet werden. Hierbei kann die (erste) mikrofluidische Vorrichtung eine übergeordnete mikrofluidische Vorrichtung sein und die mindestens zwei weiteren fluidische Vorrichtungen jeweils untergeordnete mikrofluidische Vorrichtungen sein (und *vice versa*). Darüberhinaus kann sich mindestens eine, optional können sich beide, der mindestens zwei weiteren erfindungsgemäßen mikrofluidischen Vorrichtungen selbst in beispielsweise einen untergeordneten Teil und einen übergeordneten Teil aufspalten. Hierbei kann mindestens eine weitere Kulturkammer Zellen enthalten, die keine Herzmuskelzellen sein (z.B. Zellen ausgewählt aus der Gruppe bestehend aus Leberzellen, Nierenzellen, Nervenzellen, Fettgewebe und Kombinationen hiervon) und diese in einem untergeordneten Teil der weiteren mikrofluidischen Vorrichtung enthalten sein und beispielsweise mindestens ein von der weiteren Kulturkammer verschiedener Teil in einem übergeordneten Teil der weiteren mikrofluidischen Vorrichtung. Ein übergeordneter Teil der mikrofluidischen Vorrichtung bzw. übergeordnete, weitere mikrofluidische Vorrichtungen können jeweils in Form einer Schicht ausgestaltet sein, die einen untergeordneten Teil der mikrofluidischen Vorrichtung bzw. untergeordnete, weitere mikrofluidische Vorrichtungen kontaktieren (und *vice versa*).

Es wird ferner vorgeschlagen, die erfindungsgemäße mikrofluidische Vorrichtung zur Überprüfung zu verwenden, ob eine bestimmte Substanz oder deren Metaboliten einen Einfluss auf die Aktivität von Herzmuskelzellen ausübt, bevorzugt zur Überprüfung, ob die bestimmte Substanz eine Tachykardie oder Bradykardie der Herzmuskelzellen auslöst oder eine Arrhythmie der Herzmuskelzellen bewirkt.

Darüber hinaus wird vorgeschlagen, die erfindungsgemäße mikrofluidische Vorrichtung zur Überprüfung zu verwenden, ob eine mechanische, elektrische und/oder gasbedingte Beeinflussung (z.B. über einen Oxygenator) einer Aktivität der Herzmuskelzellen einen Einfluss auf die Aktivität von biologischen Zellen ausübt, die von Herzmuskelzellen verschieden sind. Bevorzugt wird überprüft, ob und in welchem Ausmaß diese Beeinflussung einen negativen oder positiven Einfluss auf die Aktivität (Lebensfähigkeit) der biologischen Zellen ausübt.

Anhand der nachfolgenden Figuren soll der erfindungsgemäße Gegenstand näher erläutert werden, ohne diesen auf die hier dargestellten, spezifischen Ausführungsformen einschränken zu wollen.

### Bezugszeichenliste

- 1.1:: Herzmuskelzellen;
- 1.2:: Zellen eines anderen Organs (z.B. Leberzellen)
- 2:: mikrofluidischer Kanal;
- 3:: Pumpe (z.B. Mikropumpe);
- 4.X:: Detektor (z.B. Multielektrodenarray);
- 4.1:: optischer Detektor (z.B. Mikroskop);
- 4.2:: elektrischer Detektor (z.B: Multielektroden-Array ("MEA"));
- 4.3:: mechanischer Detektor (z.B: Dehnmessstreifen);
- 5:: Steuereinrichtung;
- 6.X:: Datenerfassungsgerät (z.B. zur Erfassung der Herzmuskelzellaktivität);
- 6.1:: Bradykardie;
- 6.2:: Tachykardie;
- 7.1:: Pumpenparameter zur Beeinflussung der Strömung (als Signal der Rückkopplungsschleife);
- 7.2:: Strömungsparameter zur Beeinflussung der Strömung (als Signal der Rückkopplungsschleife)
- 7.3: Gaszusammensetzung für den Oxygenator (als Signal der Rückkopplungsschleife);
- 8:: Zuführung von Substanz(en);
- 9:: Reservoir;
- 10:: Ventil;
- 11:: Drossel;
- 12:: elastische Membran;
- 13:: elektrische Vorrichtung zur Beeinflussung der Aktivität der Herzmuskelzellen;
- 14:: mechanische Vorrichtung zur Beeinflussung der Aktivität der Herzmuskelzellen;
- 15:: übergeordnete mikrofluidische Vorrichtung;
- 16.1:: erste untergeordnete mikrofluidische Vorrichtung;
- 16.2:: zweite untergeordnete mikrofluidische Vorrichtung;
- 17:: Partikel (z.B. Blutbestandteile);
- 18.X:: strukturierte Kunststofffolie;
- 19:: Vorrichtung zur Erfassung einer Strömungsgeschwindigkeit von Fluiden;
- 20:: Vorrichtung zur Erfassung des O2-Gehalts (z.B. O₂-Sensor);
- 21:: Oxygenator.
- 22:: Fluidische Schnittstelle
- 23.: Aktuierungskanal (z.B. Kanal zu einer Gasquelle)

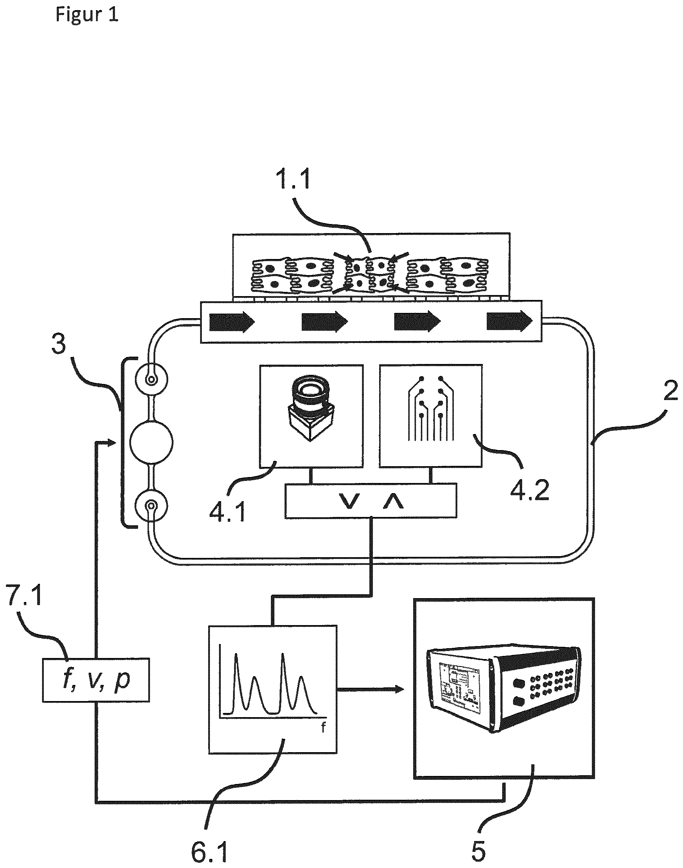

Figur 1 zeigt eine Ausgestaltung einer mikrofluidischen Vorrichtung für Zellkulturexperimente nach Anspruch 1. In dieser Ausgestaltungsform ist eine mikrofluidische Vorrichtung dargestellt, welche eine Zellkulturkammer mit Herzmuskelzellen 1.1, einen mikrofluidischen Kanal 2 und eine Pumpe 3 als fluidischen Aktor enthält. Zum Auslesen der Herzmuskelzellaktivität sind in dieser Ausgestaltung ein optischer Detektor 4.1 und/oder ein Multielektroden-Array 4.2 unterhalb der Zellkulturkammer angeordnet. Das von den Detektoren 4.x aufgenommene Signal wird von einem Datenerfassungsgerät 6.x erfasst und an eine Steuereinrichtung 5 weitergeleitet. Erfindungsgemäß werden in der Steuerung 5 die empfangenen Signale verarbeitet und die Pumpenparameter 7.1 zur Beeinflussung der Strömung angepasst.

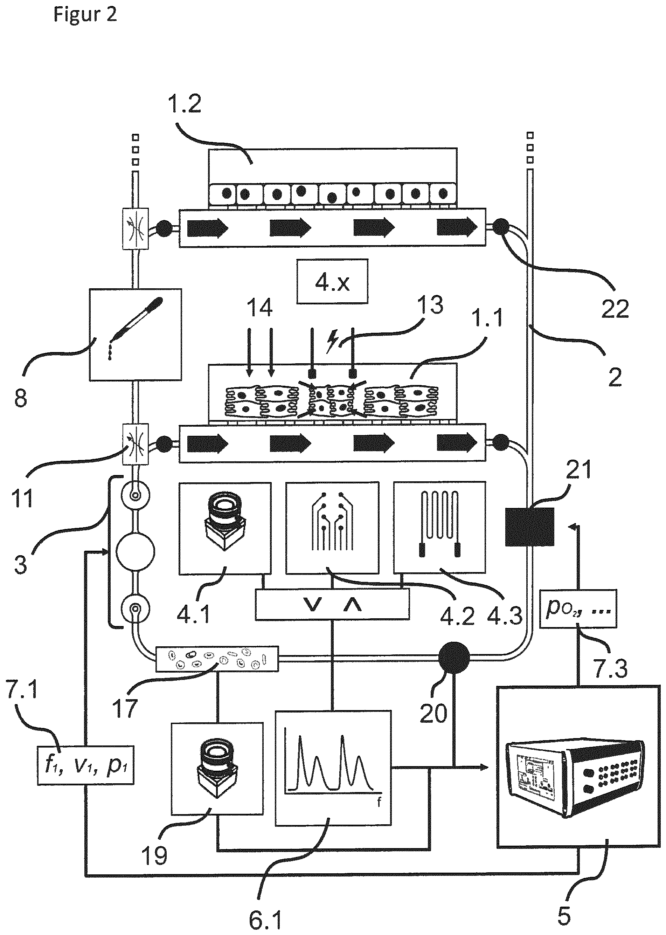

Figur 2 zeigt eine Ausgestaltung einer mikrofluidischen Vorrichtung für Zellkulturexperimente nach Anspruch 1 und Anspruch 22. In dieser Ausgestaltungsform ist eine mikrofluidische Vorrichtung dargestellt, welche mindestens einen mikrofluidischen Kanal 2 und eine Pumpe 3 als fluidischen Aktor enthält. Weiterhin sind enthalten, mehrere untergeordnete mikrofluidische Vorrichtungen mit je einer Zellkulturkammer 1.x enthaltend verschiedene Zelltypen (Herzmuskelzellen 1.1, Nierenzellen 1.2,...). Die mindestens zwei untergeordneten mikrofluidischen Vorrichtungen sind über definierte fluidische Schnittstellen 22 miteinander und der übergeordneten mikrofluidischen Vorrichtung verbunden. Weiterhin können vor jeder Kanalaufzweigung fluidische Drosseln 11 zur Volumenstromregulierung angeordnet sein. Zum Auslesen der Herzmuskelzellaktivität bzw. von physiologischen Parametern anderer Zelltypen können in dieser Ausgestaltung je ein optischer Detektor 4.1 und/oder ein Multielektroden-Array 4.2 und/oder ein Dehnmessstreifen 4.3 unterhalb der Zellkulturkammer angeordnet sein. Weiterhin können innerhalb der mikrofluidischen Vorrichtungen ein O₂-Sensor 20 sowie ein Oxygenator 21 angeordnet sein. Zur Messung der Strömungsgeschwindigkeit kann in der mikrofluidischen Vorrichtung ein optischer Detektor 19 zur Erfassung der Bewegung von zirkulierenden Partikeln oder Zellen 17 angeordnet sein. Das von den Detektoren 4.x, 19 und 20 aufgenommene Signal wird von einem Datenerfassungsgerät 6.x erfasst und an eine Steuereinrichtung 5 weitergeleitet. Erfindungsgemäß werden in der Steuerung 5 die empfangenen Signale verarbeitet und die Pumpenparameter 7.1 zur Beeinflussung der Strömung sowie die Gaszusammensetzung für den Oxygenator 7.3 angepasst.

In dieser Ausgestaltung enthält die Zellkulturkammer 1.1 weiterhin zur Beeinflussung der Aktivität der Herzmuskelzellen eine Elektrode 13 und/oder einen Aktor 14.

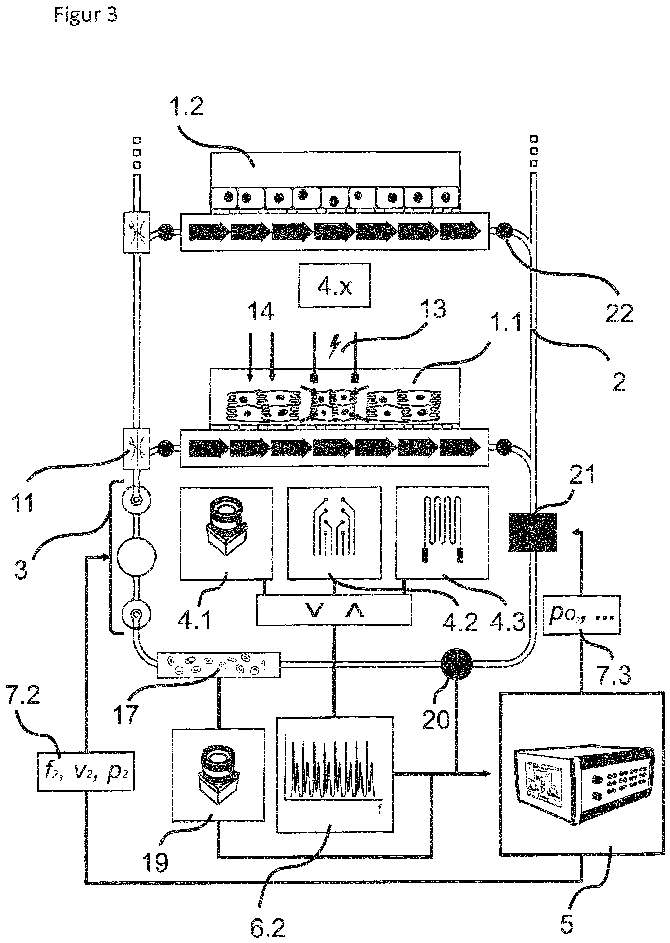

Die dargestellte mikrofluidische Vorrichtung kann Erfindungsgemäß zur Überprüfung ob eine bestimmte Substanz 8 oder deren Metaboliten einen Einfluss auf die Aktivität von Herzmuskelzellen ausübt, bevorzugt zur Überprüfung, ob die bestimmte Substanz eine Bradykardie 6.1 oder Tachykardie (Figur 3) 6.2 der Herzmuskelzellen auslöst oder eine Arrhythmie der Herzmuskelzellen bewirkt genutzt werden.

Figur 3 zeigt die mikrofluidische Vorrichtung aus Figur 2 nach Zugabe der Substanz 8. Anhand der sich durch Zugabe der Substanz ändernden Aktivität 6.2 der Herzmuskelzellen werden die Strömungsparameter 7.2 und Gaszusammensetzung 7.3 entsprechend angepasst.

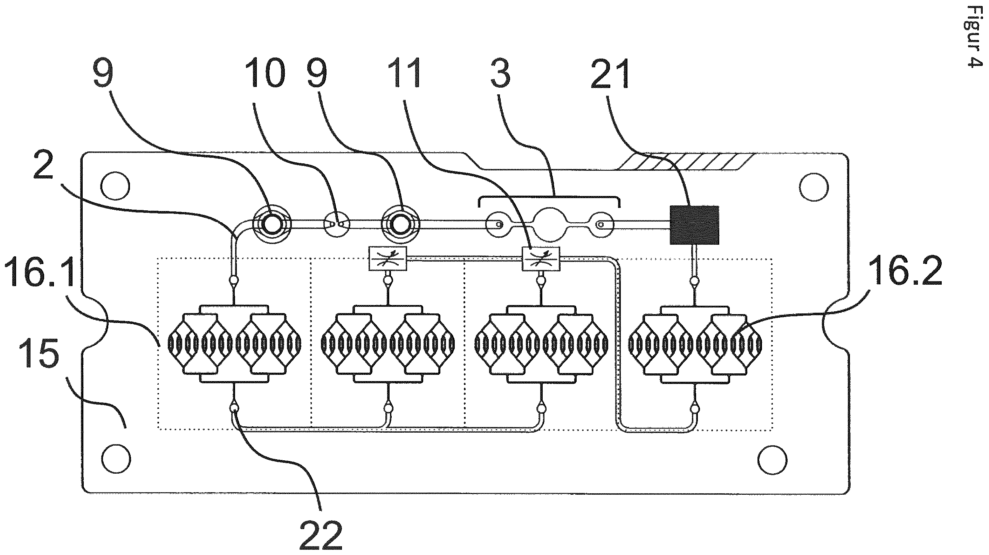

Figur 4 zeigt eine mikrofluidische Vorrichtung gemäß den Ansprüchen 1 und 22. In dieser Ausgestaltungsform bestehend aus einer übergeordneten mikrofluidischen Vorrichtung 15 enthaltend, eine Pumpe 3, mindestens ein mikrofluidischer Kanal 2, zwei Reservoire 9, ein Ventil 10, fluidische Drosseln 11 und einen Oxygenator 21. Die übergeordnete mikrofluidische Vorrichtung 15 ist über definierte fluidische Schnittstellen 22 mit mindestens einer ersten untergeordneten mikrofluidischen Vorrichtung 16.1, in diesem Fall mit mindestens einer zweiten untergeordneten mikrofluidischen Vorrichtung 16.2 verbunden.

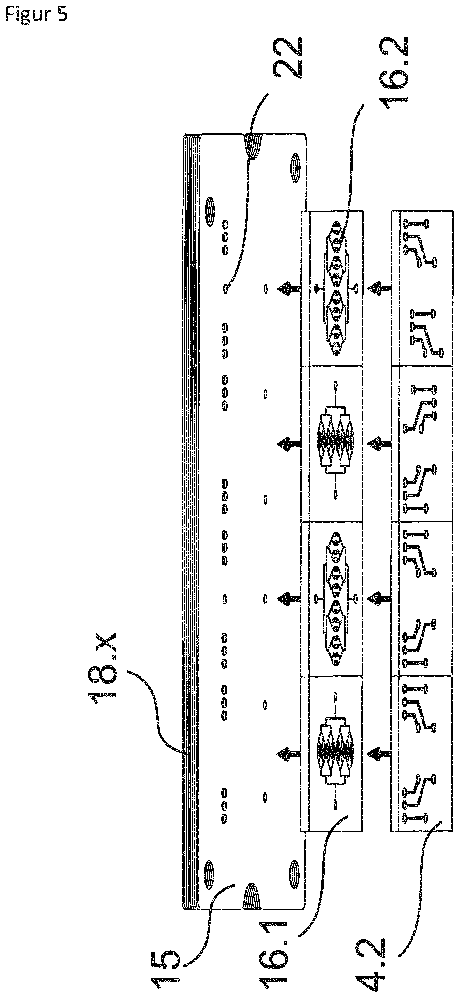

Figur 5 zeigt den Aufbau der mikrofluidischen Vorrichtung gemäß Figur 4. Enthaltend, eine übergeordnete mikrofluidische Vorrichtung 15, welche aus mehreren laminierten Kunststofffolien 18.x besteht, und mehrere untergeordnete mikrofluidische Vorrichtungen 16.1, 16.2. In dieser Ausgestaltungsform enthält die mikrofluidische Vorrichtung mehrere Multielektroden-Arrays 4.x.

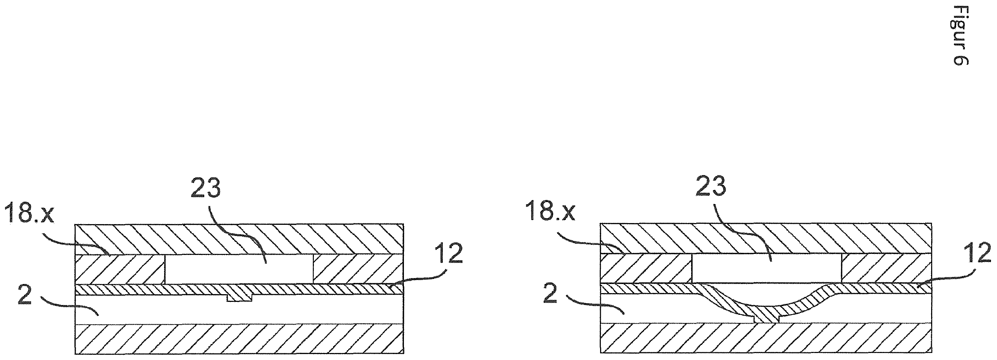

Figur 6 zeigt eine Ausgestaltungsform der fluidischen Drossel, bestehend aus mindestens drei (hier vier) laminierten Kunststofffolien 18.x, einem mikrofluidischen Kanal 2 und einer elastischen Membran 12. In dieser Ausgestaltungsform kann die elastische Membran über einen Aktuierungskanal 23 ausgelenkt werden. Figur 6 zeigt dabei den aktivierten (ausgelenkten) und den nicht aktivierten (unausgelenkten) Zustand der fluidischen Drossel.

## Patentansprüche

1. Mikrofluidische Vorrichtung, enthaltend
a) mindestens eine erste Kulturkammer enthaltend Herzmuskelzellen;
b) mindestens einen mikrofluidischen Kanal;
c) mindestens eine Pumpe zur Beförderung einer Flüssigkeit durch den mindestens einen mikrofluidischen Kanal und die mindestens eine Kulturkammer; und
d) mindestens einen Detektor, der zur Erfassung einer Aktivität der in der Kulturkammer enthaltenen Herzmuskelzellen konfiguriert ist;
wobei die mikrofluidische Vorrichtung ferner mindestens eine Steuereinrichtung enthält, die dazu konfiguriert ist, die mindestens eine Pumpe basierend auf einer von dem mindestens einen Detektor erfassten Aktivität der Herzmuskelzellen zu steuern,
wobei die mikrofluidische Vorrichtung mindestens eine Vorrichtung zur Beeinflussung einer Aktivität der in der Kulturkammer enthaltenen Herzmuskelzellen enthält,
**dadurch gekennzeichnet, dass** die Vorrichtung zur Beeinflussung einer Aktivität der in der Kulturkammer enthaltenen Herzmuskelzellen eine Elektrode enthält oder daraus besteht.

2. Mikrofluidische Vorrichtung gemäß Anspruch 1, **dadurch gekennzeichnet, dass** die mindestens eine Steuereinrichtung dazu konfiguriert ist, bei
i) einer starken Aktivität der Herzmuskelzellen den Pumpendurchsatz der mindestens einen Pumpe zu erhöhen, bevorzugt über eine Erhöhung der Pumpfrequenz und/oder des Pumpenhubs; und/oder
ii) einer schwachen Aktivität der Herzmuskelzellen den Pumpendurchsatz der mindestens einen Pumpe zu verringern, bevorzugt über eine Verringerung der Pumpfrequenz und/oder des Pumpenhubs; und/oder
iii) eine Perfusion der mindestens einen ersten Kulturkammer enthaltend Herzmuskelzellen zu verringern oder zu erhöhen, bevorzugt über ein Öffnen oder Schließen eines zur mindestens einen ersten Kulturkammer fluidisch parallel geschalteten Kurzschlusskanals; und/oder
iv) eine Perfusion mindestens einer zweiten Kulturkammer in der mikrofluidischen Vorrichtung verringern oder zu erhöhen, bevorzugt über ein Öffnen oder Schließen eines zur mindestens einen zweiten Kulturkammer fluidisch parallel geschalteten Kurzschlusskanals.

3. Mikrofluidische Vorrichtung gemäß einem der vorhergehenden Ansprüche, **dadurch gekennzeichnet, dass** die mindestens eine erste Kulturkammer Herzmuskelzellen enthält, die mindestens eine Herzmuskelfaser ausbilden, wobei die Herzmuskelzellen der mindestens eine Herzmuskelfaser bevorzugt
i) anisotrop ausgerichtet sind; und/oder
ii) biologische Zellen enthalten, die sich von Herzmuskelzellen unterscheiden, bevorzugt Zellen ausgewählt aus der Gruppe bestehend aus Fibroblasten, Endothelzellen und Kombinationen hiervon; und/oder
iii) ein Hydrogel enthalten.

4. Mikrofluidische Vorrichtung gemäß einem der vorhergehenden Ansprüche, **dadurch gekennzeichnet, dass** die mikrofluidische Vorrichtung mindestens ein Reservoir aufweist, das eine Nährlösung zur Ernährung von Herzmuskelzellen enthält, wobei
i) das Reservoir bevorzugt fluidisch mit dem mindestens einen mikrofluidischen Kanal und der mindestens einen Kulturkammer verbunden ist und besonders bevorzugt die mindestens eine Pumpe dazu konfiguriert ist, die Nährlösung zur mindestens einen Kulturkammer zu befördern, insbesondere über den mindestens einen mikrofluidischen Kanal; und/oder
ii) die mikrofluidische Vorrichtung optional dazu konfiguriert ist, bei einer starken Aktivität der Herzmuskelzellen den Austritt von Nährlösung aus dem Reservoir zu erhöhen, bevorzugt über eine Erhöhung der Pumpfrequenz, des Pumphubs und/oder durch Druckbeaufschlagung des Reservoirs; und/oder
einer schwachen Aktivität der Herzmuskelzellen den Austritt von Nährlösung aus dem Reservoir zu verringern, bevorzugt über Verringerung der Pumpfrequenz, des Pumphubs und/oder durch Druckverringerung am Reservoir.

5. Mikrofluidische Vorrichtung gemäß einem der vorhergehenden Ansprüche, **dadurch gekennzeichnet, dass** der mikrofluidische Kanal mindestens ein Ventil und/oder mindestens eine Drossel enthält, wobei das mindestens eine Ventil und/oder die mindestens eine Drossel bevorzugt
i) eine elastische Membran enthält, die bevorzugt in mindestens einer Wandung des mindestens einen mikrofluidischen Kanals angeordnet ist und besonders bevorzugt Kunststoff enthält oder daraus besteht, ganz besonders bevorzugt einen Kunststoff ausgewählt aus der Gruppe bestehend aus Thermoplast, Elastomer und Kombinationen hiervon, insbesondere einen Kunststoff ausgewählt aus der Gruppe bestehend aus PC, PET, COC, PDMS, TPE und Kombinationen hiervon; und/oder
ii) dazu geeignet ist, über eine pneumatische, thermopneumatische, elektromagnetische, elektrostatische, magnetische, chemische und/oder piezoelektrische Kraft gesteuert zu werden, wobei das Ventil und/oder die Drossel bevorzugt mit mindestens einem Aktuierungskanal und/oder mindestens einer Spannungsquelle verbunden ist;
wobei die die mindestens eine Steuereinrichtung optional ferner konfiguriert ist, basierend auf einer von dem mindestens einen Detektor erfassten Aktivität der Herzmuskelzellen, das mindestens eine Ventil und/oder die mindestens eine Drossel zu steuern, wobei die mindestens eine Steuereinrichtung bevorzugt dazu konfiguriert ist, bei
i) einer starken Aktivität der Herzmuskelzellen das mindestens eine Ventil und/oder die mindestens eine Drossel zumindest teilweise zu öffnen; und/oder
ii) einer schwachen Aktivität der Herzmuskelzellen das mindestens eine Ventil und/oder die mindestens eine Drossel zumindest teilweise zu schließen.

6. Mikrofluidische Vorrichtung gemäß einem der vorhergehenden Ansprüche, **dadurch gekennzeichnet, dass** die mikrofluidischen Vorrichtung mindestens
i) einen Oxygenator zur Oxygenierung oder Deoxygenierung der Flüssigkeit in der mikrofluidischen Vorrichtung, bevorzugt eine gaspermeable Membran und/oder eine Hohlfaser, enthält; und/oder
ii) einen O₂-Sensor zur Messung des O₂-Gehalts der Flüssigkeit in der mikrofluidischen Vorrichtung, bevorzugt einen O₂-Sensor ausgewählt aus der Gruppe bestehend aus optischer O₂-Sensor, elektrochemischer O₂-Sensor und Kombinationen hiervon, enthält;
wobei die mindestens eine Steuereinrichtung optional ferner konfiguriert ist, den Oxygenator in Abhängigkeit von einem über den O₂-Sensor gemessenen O₂-Gehalt und/oder in Abhängigkeit von der vom mindestens einen Detektor erfassten Aktivität der Herzmuskelzellen zu steuern.

7. Mikrofluidische Vorrichtung gemäß einem der vorhergehenden Ansprüche, **dadurch gekennzeichnet, dass** der Detektor einen
i) optischen Detektor, bevorzugt einen zur Messung einer Calcium-Konzentration konfigurierten optischen Detektor; und/oder
ii) elektrischen Detektor, bevorzugt ein Multi-Elektroden-Array; und/oder
iii) mechanischen Detektor, bevorzugt einen Detektor ausgewählt aus der Gruppe bestehend aus Dehnmessstreifen, Auflagekraft-Mikroskop, Federbalken und Kombinationen hiervon;
enthält oder daraus besteht.

8. Mikrofluidische Vorrichtung gemäß einem der vorhergehenden Ansprüche, **dadurch gekennzeichnet, dass** der Detektor dazu konfiguriert ist, Signale über eine Aktivität der in der mindestens einen ersten Kulturkammer enthaltenen Herzmuskelzellen an ein Datenerfassungsgerät zu senden, wobei das Datenerfassungsgerät bevorzugt dazu konfiguriert ist, die Signale in Abhängigkeit von der Zeit aufzuzeichnen und auszuwerten.

9. Mikrofluidische Vorrichtung gemäß einem der vorhergehenden Ansprüche, **dadurch gekennzeichnet, dass** die Vorrichtung zur Beeinflussung einer Aktivität der in der Kulturkammer enthaltenen Herzmuskelzellen
i) einen Aktor, besonders bevorzugt einen Aktor ausgewählt aus der Gruppe bestehend aus piezoelektrische Aktoren, elektromechanische Aktoren, pneumatische Aktoren, hydraulischen Aktoren, Oberflächenspannungsaktoren und Kombinationen hiervon; und/oder
ii) eine Vorrichtung zur Zuführung oder Abführung von Gas, bevorzugt eine Gasaustauschmembran, wobei die Vorrichtung zur Zuführung oder Abführung von Gas bevorzugt eine Quelle eines Gases ausgewählt aus der Gruppe bestehend aus (reinem) Sauerstoff, Luft (z.B. Druckluft), Stickstoff, Kohlendioxid und Kombinationen hiervon aufweist;
enthält.

10. Mikrofluidische Vorrichtung gemäß einem der vorhergehenden Ansprüche, **dadurch gekennzeichnet, dass** die mikrofluidische Vorrichtung
i) mindestens einen Zugang zur Zuführung einer chemischen, biochemischen oder biologischen Substanz enthält, wobei der Zugang optional unmittelbar in den mikrofluidischen Kanal, unmittelbar in die mindestens eine erste Kulturkammer und/oder unmittelbar in das mindestens eine Reservoir mündet; und/oder
ii) mindestens einen zweiten mikrofluidischen Kanal enthält, der bevorzugt stromaufwärts der mindestens einen ersten Kulturkammer von dem mindestens einen ersten mikrofluidischen Kanal abzweigt und stromabwärts der mindestens einen ersten Kulturkammer in den mindestens einen ersten mikrofluidischen Kanal mündet; und/oder
iii) mindestens eine zweite Kulturkammer aufweist, die biologische Zellen enthält, welche sich von Herzmuskelzellen unterscheiden, bevorzugt Zellen ausgewählt aus der Gruppe bestehend aus Leberzellen, Nierenzellen, Nervenzellen, Fettgewebe und Kombinationen hiervon, wobei die mindestens eine zweite Kulturkammer besonders bevorzugt mit der mindestens einen Kulturkammer fluidisch verbunden ist, optional über mindestens einen zweiten mikrofluidischen Kanal; und/oder
iv) mindestens einen zweiten Detektor enthält, der zur Erfassung einer Aktivität der in der zweiten Kulturkammer enthaltenen biologischen Zellen konfiguriert ist, wobei der mindestens eine zweite Detektor bevorzugt einen optischen Detektor enthält oder daraus besteht und besonders bevorzugt dazu konfiguriert ist, Signale über eine Aktivität der in der mindestens einen zweiten Kulturkammer enthaltenen biologischen Zellen an ein Datenerfassungsgerät zu senden, wobei das Datenerfassungsgerät bevorzugt dazu konfiguriert ist, die Signale in Abhängigkeit von der Zeit aufzuzeichnen und auszuwerten; und/oder
v) bevorzugt der mindestens eine mikrofluidische Kanal, die mindestens eine erste Kulturkammer und/oder mindestens ein Reservoir, Blut oder Bestandteile von Blut enthält, bevorzugt Blutzellen, besonders bevorzugt Krebszellen und/oder Immunzellen, insbesondere metastasierende Krebszellen und/oder Monocyten.

11. Mikrofluidische Vorrichtung gemäß einem der vorhergehenden Ansprüche, **dadurch gekennzeichnet, dass** die mindestens eine Steuereinrichtung eine Flussgeschwindigkeits-Messvorrichtung enthält, bevorzugt ein Particle-Image-Velocimetry-Gerät, wobei die mikrofluidische Vorrichtung besonders bevorzugt konfiguriert ist, anhand einer von der Flussgeschwindigkeits-Messvorrichtung gemessenen Flussgeschwindigkeit
i) die mindestens eine Pumpe, bevorzugt alle Pumpen, der mikrofluidischen Vorrichtung zu steuern; und/oder
ii) mindestens ein Ventil und/oder mindestens eine Drossel, bevorzugt alle Ventile und/oder alle Drosseln, der mikrofluidischen Vorrichtung, zu steuern.

12. Mikrofluidische Vorrichtung gemäß einem der vorhergehenden Ansprüche, **dadurch gekennzeichnet, dass** die mikrofluidische Vorrichtung Kunststoff enthält oder daraus besteht, wobei der Kunststoff bevorzugt
i) mindestens eine strukturierte Kunststofffolie, die mit mindestens einer weiteren, optional unstrukturierten, Kunststofffolie laminiert ist, enthält oder daraus besteht; und/oder
ii) ausgewählt ist aus der Gruppe bestehend aus Duroplasten, Thermoplasten, Elastomeren und Kombinationen hiervon, besonders bevorzugt ausgewählt ist aus der Gruppe bestehend aus PC, PET, COC, PDMS, TPE und Kombinationen hiervon; und/oder
iii) zumindest teilweise über ein Verfahren strukturiert ist, das ausgewählt ist aus der Gruppe bestehend aus Laserstrukturierung, Schneidplottern, Heißprägen, Fräsen, Thermoformen, Spritzguss, Softlithografie, 3D-Druck und Kombinationen hiervon.

13. Mikrofluidische Vorrichtung gemäß einem der vorhergehenden Ansprüche, **dadurch gekennzeichnet, dass** die Steuereinrichtung konfiguriert ist, eine Verteilung von flüssigen, festen und gasförmigen Stoffen in der mikrofluidischen Vorrichtung an einen konkreten Bedarf von Zellen oder Zellgruppen in der mikrofluidischen Vorrichtung anzupassen, bevorzugt über eine selektive Ansteuerung von der Pumpe, von mindestens einem Ventil, von mindestens einer Drossel und von einem Oxygenator, wobei die selektive Ansteuerung besonders bevorzugt über ein mathematisches Modell erfolgt, das insbesondere in der mikrofluidischen Vorrichtung gespeichert ist.

14. Mikrofluidische Vorrichtung gemäß einem der vorhergehenden Ansprüche, **dadurch gekennzeichnet, dass** die mikrofluidische Vorrichtung mindestens eine weitere mikrofluidische Vorrichtung aufweist, die fluidisch parallel oder seriell mit der mikrofluidischen Vorrichtung verbunden ist, bevorzugt über mindestens einen fluidischen Einlass und mindestens einen fluidischen Auslass, wobei die mikrofluidische Vorrichtung oder ein Teil davon besonders bevorzugt eine übergeordnete mikrofluidische Vorrichtung ist und die mindestens eine weitere fluidische Vorrichtung oder ein Teil davon eine untergeordnete mikrofluidische Vorrichtung ist, wobei ganz besonders bevorzugt ein untergeordneter Teil der mikrofluidischen Vorrichtung die mindestens eine erste Kulturkammer aufweist und ein übergeordneter Teil der Vorrichtung die mindestens eine Pumpe und/oder der mindestens einen Detektor aufweist.

15. Verwendung der mikrofluidischen Vorrichtung gemäß einem der Ansprüche 1 bis 14 zur Überprüfung, ob
i) eine bestimmte Substanz oder deren Metaboliten einen Einfluss auf die Aktivität von Herzmuskelzellen ausübt, bevorzugt zur Überprüfung, ob die bestimmte Substanz eine Tachykardie oder Bradykardie der Herzmuskelzellen auslöst oder eine Arrhythmie der Herzmuskelzellen bewirkt; und/oder
ii) eine mechanische, elektrische und/oder gasbedingte Beeinflussung einer Aktivität der Herzmuskelzellen einen Einfluss auf die Aktivität von biologischen Zellen ausübt, die von Herzmuskelzellen verschieden sind, bevorzugt zur Überprüfung, ob und in welchem Ausmaß diese Beeinflussung einen negativen oder positiven Einfluss auf die Aktivität der biologischen Zellen ausübt.

## Claims

1. A microfluidic device, comprising
a) at least one first culture chamber containing cardiomyocytes;
b) at least one microfluidic channel;
c) at least one pump for pumping a liquid through the at least one microfluidic channel and the at least one culture chamber; and
d) at least one detector, which is configured to detect an activity of the cardiomyocytes contained in the culture chamber;
wherein the microfluidic device further comprises at least one control device which is configured to control the at least one pump based on the activity of the cardiomyocytes detected by the at least one detector,
wherein the microfluidic device comprises at least one device for influencing activity of the cardiomyocytes contained in the culture chamber,
**characterized in that** the device for influencing activity of the cardiomyocytes contained in the culture chamber comprises or consists of an electrode.

2. The microfluidic device as claimed in claim 1, **characterized in that** the at least one control device is configured to
i) increase the pumping capacity of the at least one pump when there is strong activity of the cardiomyocytes, preferably by increasing the pumping frequency and/or pump lift; and/or
ii) decrease the pumping capacity of the at least one pump when there is weak activity of the cardiomyocytes, preferably by decreasing the pumping frequency and/or pump lift; and/or
iii) reduce or increase perfusion of the at least one first culture chamber containing cardiomyocytes, preferably by opening or closing a by-pass channel connected fluidically parallel to the at least one first culture chamber; and/or
iv) reduce or increase perfusion of at least one second culture chamber in the microfluidic device, preferably by opening or closing a by-pass channel connected fluidically parallel to the at least one second culture chamber.

3. The microfluidic device as claimed in one of the preceding claims, **characterized in that** the at least one first culture chamber contains cardiomyocytes, which form at least one heart muscle fiber, wherein the cardiomyocytes of the at least one heart muscle fiber preferably
i) are oriented anisotropically; and/or
ii) comprise biological cells that differ from cardiomyocytes, preferably cells selected from the group consisting of fibroblasts, endothelial cells and combinations thereof; and/or
iii) comprise a hydrogel.

4. The microfluidic device as claimed in one of the preceding claims, **characterized in that** the microfluidic device has at least one reservoir, which contains a nutrient solution for the nutrition of cardiomyocytes, wherein
i) the reservoir is connected preferably fluidically to the at least one microfluidic channel and the at least one culture chamber and especially preferably the at least one pump is configured to deliver the nutrient solution to the at least one culture chamber, in particular via the at least one microfluidic channel; and/or
ii) the microfluidic device is optionally configured to
increase the outflow of nutrient solution from the reservoir when there is strong activity of the cardiomyocytes, preferably by increasing the pumping frequency, the pump lift and/or by pressurizing the reservoir; and/or
reduce the outflow of nutrient solution from the reservoir when there is weak activity of the cardiomyocytes, preferably by decreasing the pumping frequency, the pump lift and/or by reducing the pressure on the reservoir.

5. The microfluidic device as claimed in one of the preceding claims, **characterized in that** the microfluidic channel comprises at least one valve and/or at least one throttle, wherein the at least one valve and/or the at least one throttle preferably
i) comprises an elastic membrane, which is preferably arranged in at least one wall of the at least one microfluidic channel and especially preferably comprises or consists of plastic, quite especially preferably a plastic selected from the group consisting of thermoplastic, elastomer and combinations thereof, especially a plastic selected from the group consisting of PC, PET, COC, PDMS, TPE and combinations thereof; and/or
ii) is suitable for being controlled by a pneumatic, thermopneumatic, electromagnetic, electrostatic, magnetic, chemical and/or piezoelectric force, wherein the valve and/or the throttle is preferably connected to at least one actuating channel and/or at least one voltage source;
wherein the at least one control device is optionally further configured to control the at least one valve and/or the at least one throttle, based on activity of the cardiomyocytes detected by the at least one detector, wherein the at least one control device is preferably configured to
i) at least partially open the at least one valve and/or the at least one throttle when there is strong activity of the cardiomyocytes; and/or
ii) at least partially close the at least one valve and/or the at least one throttle when there is weak activity of the cardiomyocytes.

6. The microfluidic device as claimed in one of the preceding claims, **characterized in that** the microfluidic device comprises at least
i) an oxygenator for oxygenation or deoxygenation of the liquid in the microfluidic device, preferably a gas-permeable membrane and/or a hollow fiber; and/or
ii) an O₂ sensor for measuring the O₂ content of the liquid in the microfluidic device, preferably an O₂ sensor selected from the group consisting of optical O₂ sensor, electrochemical O₂ sensor and combinations thereof;
wherein the at least one control device is optionally further configured to control the oxygenator as a function of the O₂ content measured by the O₂ sensor and/or as a function of the activity of the cardiomyocytes detected by the at least one detector.

7. The microfluidic device as claimed in one of the preceding claims, **characterized in that** the detector comprises or consists of
i) an optical detector, preferably an optical detector configured to measure the calcium concentration; and/or
ii) an electrical detector, preferably a multielectrode array; and/or
iii) a mechanical detector, preferably a detector selected from the group consisting of strain gauges, tracking force microscope, spring beam and combinations thereof.

8. The microfluidic device as claimed in one of the preceding claims, **characterized in that** the detector is configured to send signals about activity of the cardiomyocytes contained in the at least one first culture chamber to a data acquisition unit, wherein the data acquisition unit is preferably configured to record and evaluating the signals as a function of time.

9. The microfluidic device as claimed in one of the preceding claims, **characterized in that** the device for influencing activity of the cardiomyocytes contained in the culture chamber comprises
i) an actuator, especially preferably an actuator selected from the group consisting of piezoelectric actuators, electromechanical actuators, pneumatic actuators, hydraulic actuators, surface tension actuators and combinations thereof; and/or
ii) a device for supply or removal of gas, preferably a gas exchange membrane, wherein the device for supply or removal of gas preferably has a source of a gas selected from the group consisting of (pure) oxygen, air (e.g. compressed air), nitrogen, carbon dioxide and combinations thereof.

10. The microfluidic device as claimed in one of the preceding claims, **characterized in that** the microfluidic device
i) comprises at least one access for supplying a chemical, biochemical or biological substance, wherein the access optionally opens directly into the microfluidic channel, directly into the at least one first culture chamber and/or directly into the at least one reservoir; and/or
ii) comprises at least one second microfluidic channel, which preferably branches from the at least one first microfluidic channel upstream of the at least one first culture chamber, and opens into the at least one first microfluidic channel downstream of the at least one first culture chamber; and/or
iii) has at least one second culture chamber that contains biological cells, which are different from cardiomyocytes, preferably cells selected from the group consisting of liver cells, renal cells, nerve cells, fat tissue and combinations thereof, wherein the at least one second culture chamber especially preferably is connected fluidically to the at least one culture chamber, optionally via at least one second microfluidic channel; and/or
iv) comprises at least one second detector, which is configured to detect activity of the biological cells contained in the second culture chamber, wherein the at least one second detector preferably comprises or consists of an optical detector and especially preferably is configured to send signals about activity of the biological cells contained in the at least one second culture chamber to a data acquisition unit, wherein the data acquisition unit is preferably configured to record and evaluate the signals as a function of time; and/or
v) preferably the at least one microfluidic channel, the at least one first culture chamber and/or at least one reservoir, contains blood or blood constituents, preferably blood cells, especially preferably cancer cells and/or immunocytes, especially metastasizing cancer cells and/or monocytes.

11. The microfluidic device as claimed in one of the preceding claims, **characterized in that** the at least one control device comprises a flow velocity measuring device, preferably a particle image velocimetry apparatus, wherein the microfluidic device especially preferably is configured, on the basis of the flow velocity measured by the flow velocity measuring device, to
i) control the at least one pump, preferably all pumps, of the microfluidic device; and/or
ii) control at least one valve and/or at least one throttle, preferably all valves and/or all throttles, of the microfluidic device.

12. The microfluidic device as claimed in one of the preceding claims, **characterized in that** the microfluidic device comprises or consists of plastic, wherein the plastic preferably
i) comprises or consists of at least one structured plastic film, which is laminated with at least one further, optionally unstructured, plastic film; and/or
ii) is selected from the group consisting of thermosetting plastics, thermoplastics, elastomers and combinations thereof, especially preferably is selected from the group consisting of PC, PET, COC, PDMS, TPE and combinations thereof; and/or
iii) is structured at least partially by a method that is selected from the group consisting of laser structuring, cutting-plotting, hot embossing, milling, thermoforming, injection molding, soft lithography, 3D printing and combinations thereof.

13. The microfluidic device as claimed in one of the preceding claims, **characterized in that** the control device is configured to adapt the distribution of liquid, solid and gaseous substances in the microfluidic device to a concrete requirement of cells or cell groups in the microfluidic device, preferably by selective control of the pump, of at least one valve, of at least one throttle and of an oxygenator, wherein the selective control especially preferably takes place via a mathematical model, which in particular is stored in the microfluidic device.

14. The microfluidic device as claimed in one of the preceding claims, **characterized in that** the microfluidic device has at least one further microfluidic device, with parallel or serial fluidic communication with the microfluidic device, preferably via at least one fluidic inlet and at least one fluidic outlet, wherein the microfluidic device or a part thereof especially preferably is a primary microfluidic device and the at least one further fluidic device or a part thereof is a subordinate microfluidic device, wherein quite especially preferably a subordinate part of the microfluidic device has the at least one first culture chamber and a primary part of the device has the at least one pump and/or the at least one detector.

15. Use of the microfluidic device as claimed in one of claims 1 to 14 for verifying whether
i) a particular substance or metabolites thereof have an influence on the activity of cardiomyocytes, preferably for verifying whether the particular substance triggers tachycardia or bradycardia of the cardiomyocytes or gives rise to arrhythmia of the cardiomyocytes; and/or
ii) the mechanical, electrical and/or gas-induced influence of activity of the cardiomyocytes has an effect on the activity of biological cells, which are different from cardiomyocytes, preferably for verifying whether and to what extent this influence has a negative or positive effect on the activity of the biological cells.

## Revendications

1. Dispositif microfluidique, comprenant
a) au moins une première chambre de culture contenant des cellules de muscle cardiaque ;
b) au moins un canal microfluidique ;
c) au moins une pompe pour acheminer un liquide à travers le au moins un canal microfluidique et la au moins une chambre de culture ; et
d) au moins un détecteur configuré pour détecter une activité des cellules de muscle cardiaque contenues dans la chambre de culture ;
dans lequel le dispositif microfluidique comprend en outre au moins une unité de commande qui est configurée pour commander l'au moins une pompe sur la base d'une activité des cellules de muscle cardiaque détectée par l'au moins un détecteur,
dans lequel le dispositif microfluidique comprend au moins un dispositif permettant d'influencer une activité des cellules de muscle cardiaque contenues dans la chambre de culture,
**caractérisé en ce que** le dispositif permettant d'influencer 'une activité des cellules de muscle cardiaque contenues dans la chambre de culture comprend une électrode ou est constitué de la même.

2. Dispositif microfluidique selon la revendication 1, **caractérisé en ce que** l'au moins une unité de commande est configurée pour
i) augmenter le débit de pompage de la au moins une pompe en réponse à une forte activité des cellules de muscle cardiaque, de préférence en augmentant la fréquence de pompage et/ou la course de pompe ; et/ou
ii) réduire le débit de pompage de la au moins une pompe en cas de faible activité des cellules de muscle cardiaque, de préférence en réduisant la fréquence de pompage et/ou la course de pompe ; et/ou
iii) diminuer ou augmenter une perfusion de la au moins une première chambre de culture contenant des cellules de muscle cardiaque, de préférence en ouvrant ou en fermant un canal de dérivation connecté fluidiquement en parallèle à au moins une première chambre de culture ; et/ou
iv) diminuer ou augmenter une perfusion d'au moins une seconde chambre de culture dans le dispositif microfluidique, de préférence en ouvrant ou en fermant un canal de dérivation connecté fluidiquement en parallèle à au moins une seconde chambre de culture.

3. Dispositif microfluidique selon l'une des revendications précédentes, **caractérisé en ce que** la au moins une première chambre de culture contient des cellules de muscle cardiaque qui forment au moins une fibre de muscle cardiaque, dans lequel les cellules de muscle cardiaque de la au moins une fibre de muscle cardiaque sont de préférence
i) orientées de manière anisotrope ; et/ou
ii) contiennent des cellules biologiques qui sont différentes des cellules de muscle cardiaque, de préférence des cellules sélectionnées dans le groupe comprenant des fibroblastes, des cellules endothéliales et des combinaisons de celles-ci ; et/ou
iii) contiennent un hydrogel.

4. Dispositif microfluidique selon l'une des revendications précédentes, **caractérisé en ce que** le dispositif microfluidique comporte au moins un réservoir qui contient une solution nutritive destinée à nourrir les cellules de muscle cardiaque, dans lequel
i) le réservoir est de préférence relié fluidiquement au au moins un canal microfluidique et à la au moins une chambre de culture, et en particulier de préférence la au moins une pompe est configurée pour acheminer la solution nutritive vers au moins une chambre de culture, notamment via le au moins un canal microfluidique ; et/ou
ii) le dispositif microfluidique est facultativement configuré pour
augmenter le débit de solution nutritive sortant du réservoir en raison de la forte activité des cellules de muscle cardiaque, de préférence en augmentant la fréquence de pompage, la course de la pompe et/ou en pressurisant le réservoir ; et/ou
pour réduire la sortie de solution nutritive à partir du réservoir en cas de faible activité des cellules de muscle cardiaque, de préférence en réduisant la fréquence de pompage, la course de la pompe et/ou en réduisant la pression au niveau du réservoir.

5. Dispositif microfluidique selon l'une des revendications précédentes, **caractérisé en ce que** le canal microfluidique contient au moins une vanne et/ou au moins un étrangleur, dans lequel la au moins une vanne et/ou le au moins un étrangleur contiennent de préférence
i) une membrane élastique, qui est de préférence agencée dans au moins une paroi d'au moins un canal microfluidique, et plus particulièrement de préférence qui contient du plastique, de préférence un plastique choisi dans le groupe comprenant des thermoplastiques, des élastomères et des combinaisons de ceux-ci, notamment un plastique choisi dans le groupe comprenant le PC, le PET, le COC, le PDMS, le TPE et les combinaisons de ceux-ci ; et/ou
ii) qui est conçu(e) pour être commandé(e) par une force pneumatique, thermopneumatique, électromagnétique, électrostatique, magnétique, chimique et/ou piézoélectrique, dans lequel la vanne et/ou l'étrangleur sont de préférence connectés à au moins un canal d'actionnement et/ou à au moins une source de tension ;
dans lequel le au moins un dispositif de commande est en outre configuré, facultativement, pour commander au moins une vanne et/ou au moins un étrangleur en fonction de l'activité des cellules de muscle cardiaque détectée par le au moins un détecteur, dans lequel le au moins un dispositif de commande est de préférence configuré pour,
i) via une forte activité des cellules de muscle cardiaque, provoquer l'ouverture, au moins partielle, de la au moins une vanne et/ou du au moins un étrangleur ; et/ou
ii) via une faible activité des cellules de muscle cardiaque, provoquer la fermeture, au moins partielle, de la au moins une vanne et/ou du au moins un étrangleur.

6. Dispositif microfluidique selon l'une des revendications précédentes, **caractérisé en ce que** le dispositif microfluidique comprend au moins
i) un oxygénateur pour oxygéner ou désoxygéner le liquide dans le dispositif microfluidique, de préférence une membrane perméable aux gaz et/ou une fibre creuse ; et/ou
ii) comprenant un capteur d'O2 pour mesurer la teneur en O2 du liquide dans le dispositif microfluidique, de préférence un capteur d'O2 choisi dans le groupe constitué de capteurs d'O2 optiques, de capteurs d'O2 électrochimiques et de combinaisons de ceux-ci ;
dans lequel la au moins une unité de commande est facultativement configurée pour contrôler l'oxygénateur en fonction d'une teneur en O2 mesurée via le capteur O2 et/ou en fonction de l'activité des cellules de muscle cardiaque détectée par au moins un détecteur.

7. Dispositif microfluidique selon l'une des revendications précédentes, **caractérisé en ce que** le détecteur contient ou est constitué
i) d'un détecteur optique, de préférence un détecteur optique configuré pour mesurer une concentration de calcium ; et/ou
ii) d'un détecteur électrique, de préférence un réseau à plusieurs électrodes ; et/ou
iii) d'un détecteur mécanique, de préférence un détecteur choisi dans le groupe constitué de jauges de contrainte, de microscopes à force de contact, de poutres à ressort et de combinaisons de ceux-ci ;
ou en est constitué.

8. Dispositif microfluidique selon l'une des revendications précédentes, **caractérisé en ce que** le détecteur est configuré pour envoyer des signaux concernant l'activité des cellules de muscle cardiaque contenues dans la au moins une première chambre de culture à un dispositif d'acquisition de données, dans lequel le dispositif d'acquisition de données est de préférence configuré pour enregistrer et évaluer les signaux en fonction du temps.

9. Dispositif microfluidique selon l'une des revendications précédentes, **caractérisé en ce que** le dispositif est destiné à influencer l'activité des cellules de muscle cardiaque contenues dans la chambre de culture contient
i) un actionneur, de préférence un actionneur choisi dans le groupe comprenant les actionneurs piézoélectriques, les actionneurs électromécaniques, les actionneurs pneumatiques, les actionneurs hydrauliques, les actionneurs à tension superficielle et les combinaisons de ceux-ci ; et/ou
ii) un dispositif d'alimentation ou d'élimination de gaz, de préférence une membrane d'échange de gaz, dans lequel le dispositif d'alimentation ou d'élimination de gaz comprend de préférence une source de gaz choisi dans le groupe comprenant l'oxygène (pur), l'air (par exemple, l'air comprimé), l'azote, le dioxyde de carbone et des combinaisons de ceux-ci.

10. Dispositif microfluidique selon l'une des revendications précédentes, **caractérisé en ce que** le dispositif microfluidique
i) contient au moins un point d'accès pour l'introduction d'une substance chimique, biochimique ou biologique, dans lequel le point d'accès mène facultativement directement au canal microfluidique, directement à la au moins une premières chambre de culture et/ou directement dans le au moins un réservoir ; et/ou
ii) comprend au moins un second canal microfluidique, qui se ramifie de préférence en amont de la au moins une première chambre de culture à partir du au moins un premier canal microfluidique et débouche dans le au moins un premiers canal microfluidique en aval de la au moins une première chambre de culture ; et/ou
iii) au moins une seconde chambre de culture qui contient des cellules biologiques différentes des cellules de muscle cardiaque, de préférence des cellules choisies dans le groupe comprenant des cellules hépatiques, rénales, nerveuses, adipeuses et des combinaisons de celles-ci, dans lequel la au moins une seconde chambre de culture est en particulier de préférence reliée de manière fluidique à la première chambre de culture, facultativement par l'intermédiaire d'au moins un second canal microfluidique ; et/ou
iv) contient au moins un second détecteur qui est configuré pour détecter l'activité des cellules biologiques contenues dans la seconde chambre de culture, dans lequel le au moins un second détecteur est de préférence un détecteur optique et est en particulier de préférence configuré pour transmettre des signaux relatifs à l'activité des cellules biologiques contenues dans la au moins une seconde chambre de culture à un dispositif d'acquisition de données, dans lequel le dispositif d'acquisition de données est de préférence configuré pour enregistrer et analyser les signaux au fil du temps ; et/ou
v) contient de préférence au moins un canal microfluidique contenant au moins une première chambre de culture et/ou au moins un réservoir, du sang ou des composants du sang, de préférence des cellules sanguines, et plus particulièrement des cellules cancéreuses et/ou des cellules immunitaires, notamment des cellules cancéreuses métastatiques et/ou des monocytes.

11. Dispositif microfluidique selon l'une des revendications précédentes, **caractérisé en ce que** le au moins un dispositifs de commande comprend un dispositif de mesure de la vitesse d'écoulement, de préférence un dispositif de vélocimétrie par imagerie de particules, dans lequel le dispositif microfluidique est en particulier de préférence configuré, en fonction de la vitesse d'écoulement mesurée par le dispositif de mesure de la vitesse d'écoulement,
i) pour commander la au moins une pompe, de préférence de toutes les pompes, du dispositif microfluidique ; et/ou
ii) pour commander au moins une vanne et/ou au moins un étrangleur, de préférence toutes les vannes et/ou tous les étrangleurs, du dispositif microfluidique.

12. Dispositif microfluidique selon l'une des revendications précédentes, **caractérisé en ce que** le dispositif microfluidique contient ou est constitué de plastique, dans lequel plastique contient ou est constitué de préférence
i) d'au moins un film plastique structuré qui est stratifié au moins un film plastique supplémentaire, facultativement non structuré ; et/ou
ii) choisis dans le groupe comprenant des thermodurcissables, des thermoplastiques, des élastomères et des combinaisons de ceux-ci, et en particulier de préférence parmi le PC, le PET, le COC, le PDMS, le TPE et des combinaisons de ceux-ci ; et/ou
iii) est au moins partiellement structuré par un procédé choisi dans le groupe constitué de la structuration laser, des traceurs de découpe, du marquage à chaud, du fraisage, du thermoformage, du moulage par injection, de la lithographie douce, de l'impression 3D et de combinaisons de ceux-ci.

13. Dispositif microfluidique selon l'une des revendications précédentes, **caractérisé en ce que** le dispositif de commande est configuré pour adapter une distribution de substances liquides, solides et gazeuses dans le dispositif microfluidique à un besoin spécifique de cellules ou de groupes de cellules dans le dispositif microfluidique, de préférence par une commande sélective de la pompe, d'au moins une vanne, d'au moins un étrangleur et d'un oxygénateur, dans lequel la commande sélective est en particulier de préférence réalisée par un modèle mathématique stocké notamment dans le dispositif microfluidique.

14. Dispositif microfluidique selon l'une des revendications précédentes, **caractérisé en ce que** le dispositif microfluidique comprend au moins un dispositif microfluidique supplémentaire qui est relié fluidiquement en parallèle ou en série au dispositif microfluidique, de préférence via au moins une entrée fluidique et au moins une sortie fluidique, dans lequel le dispositif microfluidique, ou une partie de celui-ci, est en particulier de préférence un dispositif microfluidique maître et le dispositif microfluidique supplémentaire, ou une partie de celui-ci, un dispositif microfluidique subalterne, dans lequel de la manière la plus préférée une partie subalterne du dispositif microfluidique comprend de préférence la au moins une premières chambre de culture et une partie maître du dispositif comprend la au moins une pompe et/ou le au moins un détecteur.

15. Utilisation du dispositif microfluidique selon l'une quelconque des revendications 1 à 14 pour vérifier si
i) une substance spécifique ou ses métabolites ont un effet sur l'activité des cellules de muscle cardiaque, de préférence pour vérifier si la substance spécifique déclenche une tachycardie ou une bradycardie des cellules de muscle cardiaque ou provoque une arythmie des cellules de muscle cardiaque ; et/ou
ii) une influence mécanique, électrique et/ou gazeuse sur l'activité des cellules de muscle cardiaque a un effet sur 'une activité de cellules biologiques différentes des cellules de muscle cardiaque, de préférence pour vérifier si et dans quelle mesure cette influence a un effet négatif ou positif sur l'activité des cellules biologiques.
